# EUROPÄISCHE PATENTSCHRIFT

(11) **EP 1 421 919 B1**
(45) Veröffentlichungstag und Bekanntmachung des Hinweises auf die Patenterteilung: **20.09.2006**
(21) Anmeldenummer: 03024918.9
(22) Anmeldetag: 29.10.2003
(51) Int. Cl.: A61F 2/32

(54) **Set zur Erstellung eines künstlichen Hüftgelenks**
Set for constructing an artificial hip joint
Ensemble pour réaliser une articulation artificielle de la hanche

(30) Priorität: 05.11.2002 DE 10252123
(43) Veröffentlichungstag der Anmeldung: 26.05.2004
(73) Patentinhaber: ESKA Implants GmbH & Co., 23556 Lübeck (DE)
(72) Erfinder: Grundei, Hans, Dr. Ing., 23556 Lübeck (DE); Gradinger, Reiner, Prof. Dr., 81675 München (DE); Ascherl, Rudolf, Prof. Dr., 04439 Leipzig (DE)
(74) Vertreter: Fuchs Mehler Weiss & Fritzsche

(56) Entgegenhaltungen:
- EP-A- 0 190 093
- EP-A- 0 485 311
- EP-A- 0 808 617
- WO-A-99/60955
- DE-A- 3 710 233
- DE-C- 4 319 010
- FR-A- 2 103 145
- FR-A- 2 603 476
- US-A- 3 916 451
- US-A- 4 054 955
- US-A- 4 206 517

## Beschreibung

Die vorliegende Erfindung betrifft ein Set zur Erstellung eines künstlichen Hüftgelenkes.

Ein derartiges Set ist beispielsweise bekannt aus der DE 43 19 010 C1, dort als Beckenteilendoprothese bezeichnet. Es besteht in seiner Minimalkonfiguration aus einem Grundkörper, der eine kalottenförmige Aussparung zur Aufnahme einer künstlichen Hüftgelenkspfanne sowie einen Ansatz aufweist, in welchem mindestens eine Aufnahmebohrung ausgebildet ist. Deren Längsachse schließt mit der Polachse der Aussparung einen Winkel ein der im Bereich zwischen 20° und 30° liegt. Die Endoprothese weist darüber hinaus ein erstes Anschlusselement auf, mittels dessen eine Verbindung des Grundkörpers mit dem Darmbeinbereich des Restbeckens realisiert wird. Die Verbindung erfolgt über einen selbsthemmenden Sitz eines Zapfens des ersten Anschlusselementes in der wenigstens einen Aufnahmebohrung im Ansatz des Grundkörpers. Ziel dieses Vorschlages ist eine Endoprothese, die individuell aus einem Set von Elementen erstellt werden kann, bei der einzelne Elemente auch noch intraoperativ ausgetauscht werden können und deren Einbaulage während der Implantation noch eingestellt werden kann.

Letzteres ist jedoch nur eingeschränkt möglich, da beispielsweise der Winkel der kalottenförmigen Aussparung zu dem Ansatz unveränderlich festliegt.

Demgegenüber ist es die Aufgabe der vorliegenden Erfindung ein Set der eingangs genannten Art so weiter zu bilden, dass die daraus erstellte Endoprothese weitere Einstellmöglichkeiten als jene gemäß dem Stand der Technik bietet. Die Einstellmöglichkeiten sollen die Fehlertoleranz der Endoprothese im Falle einer nicht optimalen Implantation durch den Operateur erhöhen.

Gelöst wird die Aufgabe mit einem Set mit den Merkmalen gemäß Anspruch 1. Weitere vorteilhafte Ausgestaltungen ergeben sich aus den Unteransprüchen.

Dementsprechend weist das erfindungsgemäße Set ein Anschlusselement mit einer Basis zur Anlage an der Resektionsfläche des Darmbeinbereiches des Restbeckens, von der wenigstens ein nach caudal weisender Schuh zum intramedullären Einsatz in den Darmbeinbereich abragt, eine an dem Anschlusselement befestigbare Gelenkkugel, eine Gelenkpfanne, in welche die Gelenkkugel setzbar ist, derart, dass die Gelenkpfanne Verschwenkbewegungen ausführen kann, und einen am Polbereich der Gelenkpfanne angeordneten Adapter zur Herstellung einer dauerhaften, aber lösbaren Verbindung mit einer Femurstielendoprothese auf.

Mit dem Restbecken verbunden ist also die Gelenkkugel, wohingegen der bewegliche Teil des künstlichen Hüftgelenkes die Gelenkpfanne darstellt.

Gemäß einer vorteilhaften Weiterbildung ist die Verbindung zwischen dem Anschlusselement und der Gelenkkugel über einen konischen Klemmsitz eines Klemmkonus in einer in der Gelenkkugel angeordneten Klemmhülse herstellbar. Dabei ist der Klemmkonus von dorsal nach ventral gesehen in einem Winkel im Bereich von 25° bis 75°, vorzugsweise 35°, und von medial nach lateral in einem Winkel δ im Bereich 0° bis 10°, besonders bevorzugt 5°, geneigt. Diese Winkelbereiche sind angegeben, obwohl die absoluten Maße eines Beckens von einem Patienten zum anderen Patienten variieren. In nur geringeren Grenzen variieren hingegen die relativen Maße und Winkellagen, so dass diese Winkelangaben gemacht werden können.

Gemäß einer weiteren vorteilhaften Weiterbildung ist vorgesehen, dass die Achse der konischen Klemmhülse in der Gelenkkugel mit der Durchmesserlinie einem Winkel im Bereich von 7° bis 15° einschließt und das die konische Klemmhülse mit einem Versatz von 1 mm bis 4 mm gegenüber der Durchmesserlinien dezentriert angeordnet ist. Dies gestattet eine Feineinstellung der Lage zwischen Anschlusselement und Gelenkkugel.

Vorzugsweise ist die konische Klemmhülse der Gelenkkugel von einem Bund eingefasst, der wenigstens von einer radialen Bohrung durchsetzt ist, durch welche nach Herstellung des konischen Klemmsitzes der Gelenkkugel auf dem Klemmkonus eine Arretierungsschraube schraubbar ist, derart, dass die Arretierungsschraube in eine am Fuß des Klemmkonus umlaufende Kerbrille greift. Diese Maßnahme dient zur Sicherung des Klemmsitzes der Kugel am Anschlusselement.

Gemäß einer vorteilhaften Ausführungsform besteht der Adapter aus einem auf einen Ansatz im Polbereich der Gelenkpfanne setzbaren und dort arretierbaren Winkeladapter, der so ausgebildet ist, dass ein konischer Klemmzapfen oder eine konische Klemmhülse als Verbindungsmittel zu der Femurstielendoprothese vorgesehen ist, der bzw. die eine Hauptachse aufweisen, die in einem Winkel zwischen 5° und 15° zu Polachse der Gelenkpfanne steht.

Wie die Verbindung zwischen Anschlusselement und Gelenkkugel kann auch die Verbindung zwischen dem Adapter und der Femurstielendoprothese ausgebildet sein, das heißt, dass eine Klemmhülse exzentrisch in der Femurstielendoprothese vorgesehen ist, wenn der Adapter einen Klemmkonus aufweist. Damit ist eine Feinverstellbarkeit der Lage des Adapters in bezug auf die Femurstielendoprothese gegeben. Diese Feineinstellbarkeit macht die aus dem Set erstellte Hüftgelenksprothese fehlertolerant gegenüber etwaigen Implantationsungenauigkeiten.

Gemäß einer noch weiteren vorteilhaften Weiterbildung ist vorgesehen, dass die Basiskante der Gelenkpfanne in dem ventral zugewandten Bereich in einem Segment von bis zu 90° eine geschwungene Ausnehmung aufweist. Hierdurch wird die Beugefähigkeit des Gelenkes erhöht, was insbesondere dann eine Rolle spielt, wenn der mit der Prothese versorgte Patient sitzen möchte. Die Ausnehmung gestattet eine stärkere Beugung des Femurs nach ventral, die so trotz der engen Platzverhältnisse realisiert werden kann.

Die Gelenkpfanne besteht vorzugsweise aus einer Metallschale mit einem darin eingesetzten Inlay. Das Inlay kann aus hochverdichtetem Polyethylen oder aus einem körperverträglichen Kompositwerkstoff bestehen.

Das Inlay wird in der Metallschale dadurch arretiert, dass das Inlay einen umlaufenden Federring aufweist, der in eine entsprechende umlaufende Rastnut in der Metallschale rastbar ist.

Die Erfindung wird beispielhaft anhand eines Ausführungsbeispiels gemäß den Zeichnungsfiguren näher erläutert.

Hierbei zeigt:
- Fig. 1: das Model eines resezierten Beckens mit einer Beckenprothese, die aus dem Set erstellt wurde.
- Fig. 2: die Bestandteile des Sets in Explosionsdarstellung,
- Fig. 3: die Vergrößerung der Einzelheit Z in Fig. 1, und

Nachfolgend bezeichnen gleiche Bezugszeichen gleiche Teile.

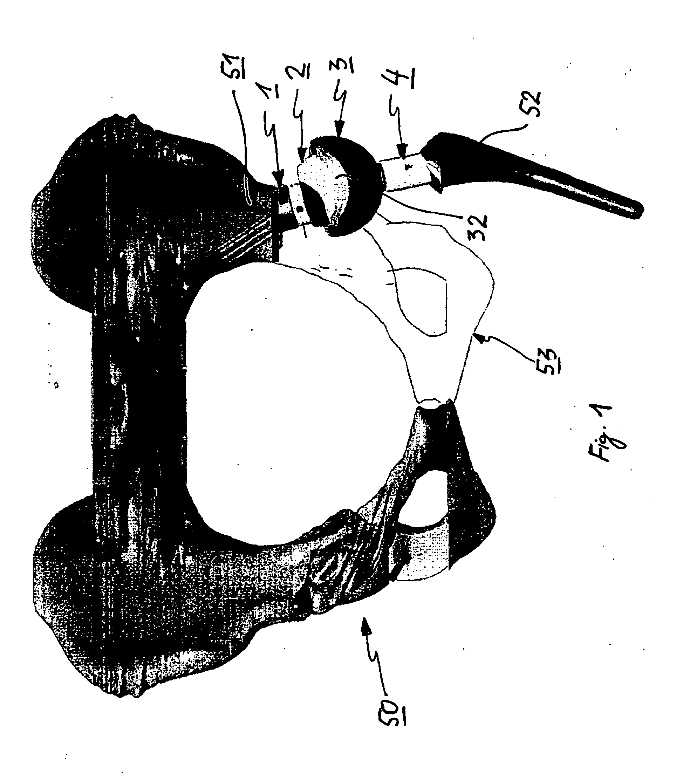

Eine erste Übersicht verschafft Fig. 1. Diese zeigt ein Beckenmodell 50. Der Darmbeinbereich 53 ist vorliegend nur angedeutet. Die Darstellung soll die Situation veranschaulichen nach Durchführung der Resektion im Darmbeinbereich, wonach hiervon nur noch ein Darmbeinstumpf 51 verbleibt. An dem Darmbeinstumpf ist nun ein künstliches Hüftgelenk angebracht. Die Verbindung zwischen dem Darmbeinstumpf 51 und dem künstlichen Hüftgelenk wird geschaffen durch ein Anschlusselement 1. Mit dem Anschlusselement 1 ist eine Gelenkkugel 2 verbunden. Diese Gelenkkugel 2 liegt in einer Gelenkpfanne 3. Gut erkennbar ist bei dieser Darstellung, dass die Basiskante der Gelenkpfanne 3 in einem ventral zugewandten Bereich in einem Segment von vorliegend ca. 60° eine geschwungene Ausnehmung 32 aufweist. Dies gestattet eine größere Verschwenkbewegung der Pfanne 3, was unter anderem dann eine Rolle spielt, wenn sich der Patient setzen möchte.

Mit der Gelenkpfanne 3 ist ein Adapter 4 verbunden. Mit diesem wiederum ist eine Femurstielendoprothese 52 verbunden.

Der Detailaufbau der Prothese ergibt sich aus Fig. 2. Darin ist ein Anschlusselement 1 zu erkennen. Das Anschlusselement 1 weist eine Basis 11 auf, die auf der Resektionsfläche des Darmbeinstumpfes 51 zu liegen kommt. Nach caudal hin, also in Fig. 2 nach links von der Basis 11 abragend ist ein Schuh 12. Dieser Schuh 12 wird intramedullär in den Darmbeinstumpf gesetzt und dient zur Vergrößerung der Lastverteilungsfläche.

Zur anderen Seite der Basis 11 ist ein Klemmkonus 14 angeformt, welcher als Verbindungsmittel mit der Gelenkkugel 2 dient. Der Klemmkonus 14 steht zur Hauptachse der Basis 11 in einem Winkel y, der im Bereich von 25° bis 75° liegt.

In der Gelenkkugel 2 ist eine konische Klemmhülse (nicht dargestellt) vorgesehen, in welche der Klemmkonus 14 des Anschlusselementes gesetzt werden kann, wobei dann eine konische Klemmverbindung entsteht. Die Klemmhülse der Gelenkkugel 2 ist eingefasst von einem Bund 21. In diesem Bund ist wenigstens eine Bohrung 22 vorgesehen, in welche nach Herstellung der konischen Klemmverbindung zwischen Anschlusselement und Gelenkkugel 2 ein Schraube (nicht dargestellt) gesetzt und mit dieser verschraubt werden kann, derart, dass die Schraube in eine am Fuß des Klemmkonus 14 umlaufende Kerbrille 15 greift. Dies ist eine Sicherungsmaßnahme gegen eine Lockerung des konischen Klemmsitzes des Klemmkonus 14 in der Klemmhülse in der Gelenkkugel 2.

Auf der Gelenkkugel 2 ist ein flächiger Anschliff 23 vorgesehen. Dieser Anschliff 23 ermöglicht es bei der Zusammenstellung von Gelenkkugel 2 und Gelenkpfanne 3, dass die Gelenkpfanne 3 über den Äquator der Gelenkkugel 2 greift. Ohne Anschliff 23 könnte diese Antiluxationshilfe nicht vorgesehen sein.

Die Gelenkpfanne 3 besteht vorliegend aus einer Metallschale 33, welche innen mit einem Inlay 34 ausgekleidet ist. Sie weist im Polbereich einen Ansatz 31 auf, welcher für die Verbindung mit dem Adapter 4 dient.

Ihre Basiskante B ist in dem nach ventral weisenden Bereich mit einer geschwungenen Ausnehmung 32 versehen, wie sich in Fig. 2 aus dem nicht geschnittenen, unteren Teil ergibt. Diese Ausnehmung 32 gestattet eine größere Verschwenkung in den ventralen Bereich.

Auf den Ansatz 31 der Gelenkpfanne 3 wird der Adapter 4 gesetzt und dort arretiert, und zwar mittels Schrauben (nicht dargestellt), welche durch die Bohrung 42 gesetzt werden.

Als Verbindungselement zu einer Femurstielendoprothese (nicht dargestellt) dient ein konischer Klemmzapfen 41, der mit einer entsprechend ausgebildeten konischen Klemmhülse in der Femurstielendoprothese zusammenwirkt.

Der Adapter 4 ist vorliegend als Winkeladapter ausgeführt, was nichts anderes bedeutet, als dass die Hauptachse H des konischen Klemmzapfens 41 gegenüber der Polachse P der Gelenkpfanne 2 geneigt ist in einem Winkel zwischen 5° bis 15°.

Die konische Klemmverbindung zwischen dem Anschlusselement 1 und der Gelenkkugel 2 ist besonders bevorzugt als sogenannte Varioverbindung ausgeführt. Dies bedeutet, dass die Achse der konischen Klemmhülse in der Gelenkkugel 2 mit der Durchmesserlinie D einen Winkel α im Bereich von 7° < α < 15° einschließt. Die konische Klemmhülse in der Gelenkkugel 2 ist mit einem Versatz von 1 mm bis 4 mm gegenüber der Durchmesserlinie D dezentriert angeordnet.

Die Verbindung zwischen dem Adapter 4 und der Femurstielendoprothese ist besonders bevorzugt ebenfalls als Varioverbindung ausgeführt.

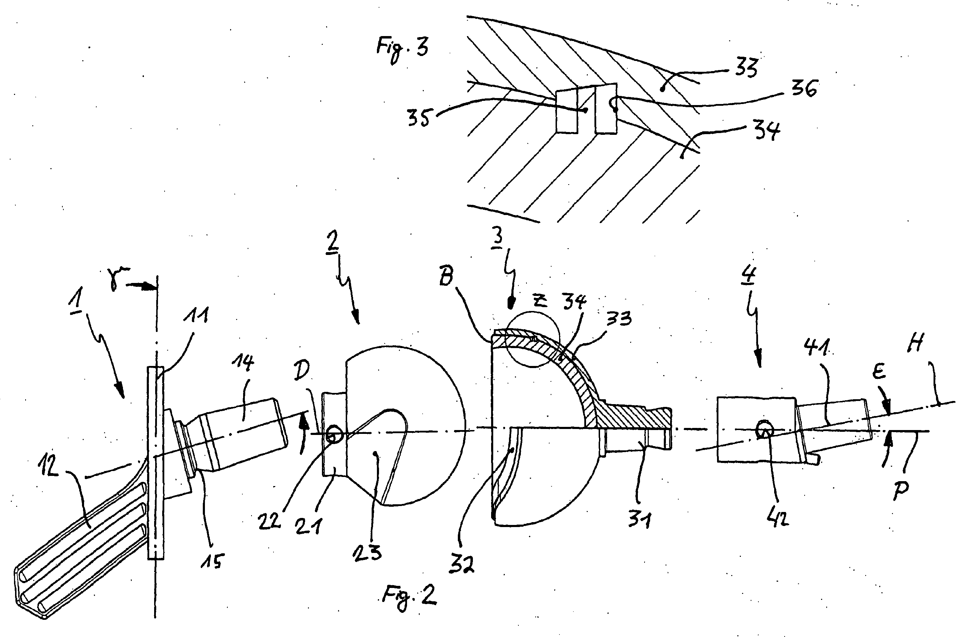

Fig. 3 zeigt noch eine Einzelheit, und zwar die Vergrößerung der Einzelheit Z in Fig. 2. Darin ist die Art und Weise dargestellt, wie das Inlay 34 in der Metallschale 33 arretiert ist.

Hierzu weist das Inlay 34 einen umlaufenden Federring 35 auf. An entsprechender Stelle weist die Metallschale 33 eine entsprechend umlaufende Rastnut 36 auf. Bei Einsatz des Inlays 34 in die Metallschale 33 rastet der Federring 35 in die Rastnut 36 ein und hält das Inlay sicher in dieser Lage, die sich auch unter Belastung des Gelenkes nicht verändert.

### Bezugszeichenliste

- 1: Anschlusselement
- 2: Gelenkkugel
- 3: Gelenkpfanne
- 4: Adapter
- 11: Basis
- 12: Schuh
- 14: Klemmkonus
- 15: Kerbrille
- 21: Bund
- 22: Radiale Bohrung
- 23: Anschliff
- 31: Ansatz
- 32: Geschwungene Ausnehmung
- 33: Metallschale
- 34: Inlay
- 35: Umlaufender Federring
- 36: Umlaufende Rastnut
- 41: Konischer Klemmzapfen
- 42: Bohrung
- 50: Restbecken
- 51: Darmbeinbereich
- 52: Femurstielendoprothese
- 53: Resezierter Beckenknochen

## Patentansprüche

1. Set zur Erstellung eines künstlichen Hüftgelenkes, aufweisend
- ein Anschlusselement (1) mit einer Basis (11) zur Anlage an der Resektionsfläche des Darmbeinbereiches des Restbeckens, von der aus wenigstens ein nach caudal weisender Schuh (12) zum intramedullären Einsatz in den Darmbeinbereich abragt,
- eine an dem Anschlusselement (1) befestigbare Gelenkkugel (2),
- eine Gelenkpfanne (3), in welche die Gelenkkugel (2) setzbar ist, derart, dass die Gelenkpfanne (3) Verschwenkbewegungen ausführen kann, und
- einen am Polbereich der Gelenkpfanne (3) angeordneten Adapter (4) zur Herstellung einer dauerhaften, aber lösbaren Verbindung mit einer Femurstielendoprothese.

2. Set nach Anspruch 1, bei dem die Verbindung zwischen dem Anschlusselement (1) und der Gelenkkugel (2) über einen konischen Klemmsitz eines Klemmkonus (14) in einer in der Gelenkkugel (2) angeordneten Klemmhülse herstellbar ist, wobei der Klemmkonus (14) von dorsal nach ventral gesehen in einem Winkel γ im Bereich 25° < γ < 75°, vorzugsweise γ = 35°, und von medial nach lateral in einem Winkel δ im Bereich 0° < δ < 10°, vorzugsweise δ = 5°, geneigt ist.

3. Set nach Anspruch 2, bei dem die Achse der konischen Klemmhülse in der Gelenkkugel (2) mit der Durchmesserlinie (D) einen Winkel α im Bereich von 7° < α < 15° einschließt und die konische Klemmhülse mit einem Versatz von 1 bis 4 mm gegenüber der Durchmesserlinie dezentriert angeordnet ist.

4. Set nach Anspruch 2 oder 3, bei dem die konische Klemmhülse der Gelenkkugel (2) von einem Bund (21) eingefasst ist, den wenigstens eine radiale Bohrung (22) durchsetzt, durch welche nach Herstellung des konischen Klemmsitzes der Gelenkkugel (2) auf den Klemmkonus (14) eine Arretierungsschraube schraubbar ist, derart, dass die Arretierungsschraube in eine am Fuß des Klemmkonus (14) umlaufende Kerbrille (15) greift.

5. Set nach einem der Ansprüche 1 bis 4, bei dem der Adapter (4) aus einem auf einen Ansatz (31) im Polbereich der Gelenkpfanne (3) setzbaren und dort arretierbaren Winkeladapter besteht, der so ausgebildet ist, dass ein konischer Klemmzapfen (41) oder eine konische Klemmhülse als Verbindungsmittel zu der Femurstielendoprothese vorgesehen ist, der bzw die eine Hauptachse (H) aufweisen, die in einem Winkel ε zwischen 5° und 15° zur Polachse (P) der Gelenkpfanne (2) steht.

6. Set nach einem der Ansprüche 1 bis 5, bei dem die Basiskante (B) der Gelenkpfanne (3) in dem ventral zugewandten Bereich in einem Segment von bis zu 90° eine geschwungene Ausnehmung (32) aufweist.

7. Set nach einem der Ansprüche 1 bis 6, bei dem die Gelenkpfanne (3) aus einer Metallschale (33) mit darin eingesetztem Inlay (34) aus hoch verdichtetem Polyethylen besteht.

8. Set nach einem der Ansprüche 1 bis 6, bei dem die Gelenkpfanne (3) aus einer Metallschale (33) mit darin eingesetztem Inlay (34) aus einem körperverträglichen Kompositwerkstoff besteht.

9. Set nach Anspruch 7 oder 8, bei dem das Inlay (34) einen umlaufenden Federring (35) aufweist, der in eine entsprechende umlaufende Rastnut (36) in der Metallschale (33) rastbar ist.

## Claims

1. Set for the construction of an artificial hip joint comprising
- a connector element(1) with a basis (11) for the attachment to the resection surface of the ilium area of the pelvis, from which at least one caudal directed shoe (12) points to the intramedullarly inset in the ilium area,
- a ball joint (2) fastenable to the connector element (1),
- a joint socket (3) in which the ball joint (2) can be set in such a way that the joint socket (3) can perform deviating movements, and
- an adapter (4) arranged for the pole area of the socket joint(3) for the creation of a durable but detachable connection with a femur shaft prosthesis.

2. Set according to claim 1, in which the connection between the connector element(1) and the ball joint (2) can be produced over a conical clamp seat of a clamping cone (14) in a clamping sleeve arranged in the socket joint (2) wherein the clamping cone (14) is inclined from the dorsal to the ventral at an angle γ in the range of 25° < γ < 75°, preferably γ = 35°, and from the medial to the lateral at an angle *δ* in the range of 0° < δ < 10°, preferably δ = 5°.

3. Set according to claim 2, in which the axis of the clamping sleeve in the socket joint (2) encases an angle α in the range of 7° < α < 15° with the diameter line (D), and the conical clamping sleeve is arranged with an offset of 1 to 4 mm decentered against the diameter line.

4. Set according to claims 2 or 3, wherein the conical clamping sleeve of the ball joint (2) is bound with a collar (21) which pushes through at least one radial drilling (22), through which an adjustable screw can be screwed into the clamping cone (14) after creating the conical clamp seat of the socket joint (2) on the clamping cone in such a way that the adjustable screw grips into a surrounding groove (15) at the foot of the clamping cone (14).

5. Set according to one of the claims 1 through 4, in which the adapter (4) consists of an angle adapter that can be lockably placed onto a lug (31) in the pole area of the socket joint(3) which is designed in such a way that a conical clamping stud (41) or a conical clamping sleeve is designated as connecting means to the femur shaft prosthesis, which shows a main axis (H) which stands at an angle ε between 5° and 15° to the Pole axis (P) of the socket joint (2).

6. Set according to claims 1 through 5, in which the swivel base (B) of the socket joint (3) exhibits a curved cutout (32) in a segment up to 90° in the ventrally turned area.

7. Set according to claims 1 through 6, in which the socket joint (3) consists of a metal socket (33) with an inset inlay (34) made of high density polyethylene.

8. Set according to claims 1 through 6, in which the socket joint (3) consists of a metal socket (33) with an inset inlay (34) made of a biocompatible composite material.

9. Set according to claims 7 or 8, in which the inlay (34) shows a surrounding lockwasher (35) being engageable into a matching, surrounding groove (36) in the metal socket (33).

## Revendications

1. Ensemble d'éléments pour la réalisation d'une articulation artificielle de la hanche, comprenant
- un élément de raccord (1) comportant une base (11) de fixation à la surface de résection de la zone de l'ilion du bassin, la surface présentant au moins un sabot à orientation caudale utilisable de façon intra médullaire dans la zone de l'ilion,
- une tête d'articulation (2) à fixer à l'élément de raccord (1),
- une cavité glénoïde (3) dans laquelle la tête d'articulation peut être logée de manière à permettre des mouvements de rotation de ladite cavité, et
- un adaptateur (4) placé dans la zone polaire de la cavité glénoïde (3) pour la réalisation d'un raccord solide mais amovible à une endoprothèse de l'extrémité de la tige du fémur.

2. Ensemble d'éléments selon la revendication 1, dans lequel la connexion entre l'élément de raccord (1) et la tête d'articulation (2) est réalisable au-dessus d'un siège de serrage conique d'un cône de serrage (14) dans une coque de serrage placée dans la tête d'articulation (2), et où le cône de serrage (14) penche selon un angle γ situé dans une plage 25° < γ < 75°, de préférence γ = 35°, en vue de la face dorsale vers la face ventrale, et selon un angle δ situé dans la plage 0° < δ < 10°, de préférence δ = 5°, en vue de la face médiale vers la face latérale.

3. Ensemble d'éléments selon la revendication 2, dans lequel l'axe de la coque de serrage conique dans la tête d'articulation (2) présente un angle α avec le diamètre (D) situé dans la plage 7° < α < 15°, et la coque de serrage conique est placée de façon décentrée avec un décalage de 1 à 4 mm par rapport au diamètre.

4. Ensemble d'éléments selon la revendication 2 ou 3, dans lequel la coque de serrage conique de la tête d'articulation (2) est entourée d'un lien (21) présentant au moins une perforation radiale (22), au travers de laquelle il est possible de visser une vis de fixation après la réalisation du siège de serrage conique de la tête d'articulation sur le cône de serrage (14), et de manière à ce que la vis de fixation soit en prise dans une gorge d'entaille (15) entourant la base du cône de serrage (14).

5. Ensemble d'éléments selon une des revendications 1 à 4, dans lequel l'adaptateur (4) est constitué par un adaptateur angulaire pouvant être placé et ancré sur un épaulement (31) situé dans la zone polaire de la cavité glénoïde (3), l'adaptateur étant conçu de manière à ce que l'on puisse prévoir un tenon de serrage conique (41) ou une coque de serrage conique en tant que moyens de connexion à l'endoprothèse de l'extrémité de la tige du fémur, et le tenon de serrage conique ou la coque présente un axe principal (H) situé dans un angle ε de 5° à 15° par rapport à l'axe polaire (P) de la cavité glénoïde (2).

6. Ensemble d'éléments selon l'une des revendications 1 à 5, dans lequel l'angle de base (B) de la cavité glénoïde (3) présente un creux incurvé (32) sur un segment jusqu'à 90° dans la zone tournée vers la face ventrale.

7. Ensemble d'éléments selon l'une des revendications 1 à 6, dans lequel la cavité glénoïde (3) est composée d'une enveloppe métallique (33) renfermant une incrustation (34) en polyéthylène haute densité.

8. Ensemble d'éléments selon l'une des revendications 1 à 6, dans lequel la cavité glénoïde (3) est composée d'une enveloppe métallique (33) renfermant une incrustation (34) réalisée dans un matériau composite toléré par l'organisme.

9. Ensemble d'éléments selon la revendication 7 ou 8, dans lequel l'incrustation (34) présente une rondelle bombée (35) sur le pourtour, qui peut être maintenue dans un écrou de fixation (36) annulaire correspondant dans l'enveloppe métallique (33).
